# EUROPÄISCHE PATENTSCHRIFT

(11) **EP 3 138 030 B1**
(45) Veröffentlichungstag und Bekanntmachung des Hinweises auf die Patenterteilung: **28.07.2021**
(21) Anmeldenummer: 15722679.6
(22) Anmeldetag: 29.04.2015
(51) Int. Cl.: G16H 10/20, G16H 10/60

(54) **PATIENTENREKRUTIERUNGSSYSTEM UND PATIENTENREKRUTIERUNGSVERFAHREN**
PATIENT RECRUITMENT SYSTEM AND PATIENT RECRUITMENT METHOD
SYSTÈME DE RECRUTEMENT DE PATIENTS ET PROCÉDÉ DE RECRUTEMENT DE PATIENTS

(30) Priorität: 30.04.2014 DE 102014106112
(43) Veröffentlichungstag der Anmeldung: 08.03.2017
(73) Patentinhaber: Clinerion Ltd., 4053 Basel (CH)
(72) Erfinder: WALTER, Andreas, 76534 Baden-Baden (DE); CLAESSON, Ulf, CH-8907 Wettswil (CH); ARONSKY Dominik, CH-8803 Rueschlikon (CH); BODENMANN, Bernhard, CH-4102 Binningen (CH)
(74) Vertreter: Daub, Thomas
(86) Internationale Anmeldenummer: PCT/EP2015/059415
(87) Internationale Veröffentlichungsnummer: WO 2015/166005

(56) Entgegenhaltungen:
- CA-A1- 2 337 665
- DE-A1-102007 026 802
- US-A1- 2012 191 749
- US-A1- 2013 191 161
- US-A1- 2013 304 542
- None

## Beschreibung

### Stand der Technik

Die Erfindung betrifft ein Patientenrekrutierungssystem nach Anspruch 1 und ein Patientenrekrutierungsverfahren nach dem Anspruch 17.

Es ist bereits ein Patientenrekrutierungssystem mit zumindest einem Patientenrekrutierungsserver, der zumindest eine Suchroutine aufweist, die dazu vorgesehen ist, Patientendatensätze anhand von Rekrutierungsmerkmalen zu suchen, vorgeschlagen worden. Aus den Veröffentlichungen DE 10 2007 026 802 A1, US 2013 / 304542 A1, CA 2 337 665 A1 und US 2012 / 191749 A1 sind bereits Patientenrekrutierungssysteme zur Rekrutierung von Patienten für klinische Studien bekannt. Aus der US 2013 / 191161 A1 ist zudem ein Verwaltungssystem für elektronische Gesundheitsakten bekannt. Aufgrund der besonderen Sensibilität von Patientendaten besteht das Bedürfnis die Sicherheit der bekannten Systeme gegenüber einem unbefugten Abgreifen von Daten, z.B. durch Hacking o.dgl., wesentlich zu erhöhen.

Die Aufgabe der Erfindung besteht insbesondere darin, eine gattungsgemäße Vorrichtung mit vorteilhaften Eigenschaften hinsichtlich einer Datensicherheit bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Offenbarung der Erfindung

Die Erfindung geht aus von einem Patientenrekrutierungssystem, insbesondere zur Rekrutierung von Patienten im medizinischen und/oder veterinärmedizinischen Bereich, mit zumindest einem Patientenrekrutierungsserver, der zumindest eine Suchroutine aufweist, die dazu vorgesehen ist, Patientendatensätze anhand von Rekrutierungsmerkmalen zu suchen.

Erfindungsgemäß wird vorgeschlagen, dass der Patientenrekrutierungsserver eine Benachrichtigungsroutine aufweist, die dazu vorgesehen ist, wenigstens zu einem mittels der Rekrutierungsmerkmale gefundenen Patientendatensatz eine Rekrutierungsnachricht zu versenden.

Unter einem "Patientenrekrutierungsserver" soll insbesondere ein, insbesondere zentral angeordneter, Server und/oder eine, insbesondere zentrale, Kontrollinstanz verstanden werden, der zur Patientenrekrutierung in mehreren Gesundheitsinstitutionen, insbesondere Krankenhäusern, vorgesehen ist und hierzu insbesondere in zumindest einem Betriebszustand, insbesondere bei einer Anfrage einer Patientendatenaufberei tungseinheit, kodierte Suchanfragen an Patientendatenaufbereitungseinheiten überträgt. Vorzugsweise ist der Patientenrekrutierungsserver als ein dedizierter Server ausgebildet, könnte aber alternativ auch als ein virtueller Server, als mehrere verteilt angeordnete Server oder als ein Server in einer Cloud ausgebildet sein. Der Patientenrekrutierungsserver ist außerhalb der Intranets jeweiliger Gesundheitsinformationssysteme, beispielsweise jeweiliger Krankenhausinformationssysteme, Arztpraxisinformationssysteme, Krebsregister oder ähnlicher Systeme, welche irgendwelche Patienten- oder patientenverwandte Daten beinhalten, einer Gesundheitsinstitution, insbesondere eines Krankenhauses, angeordnet. Vorzugsweise ist der Patientenrekrutierungsserver dazu vorgesehen, Patienten für einen, einem Fachmann als sinnvoll erscheinenden Vorgang, vorteilhaft jedoch für klinische Studien und/oder für eine Organtransplantation, zu rekrutieren. Vorzugsweise kommunizieren die Patientendatenaufbereitungseinheiten des Patientenrekrutierungssystems und der Patientenrekrutierungsserver mittels einer, dem Fachmann als sinnvoll erscheinenden Server-to-Server Kommunikation. Vorzugsweise kommunizieren die Patientendatenaufbereitungseinheiten verschlüsselt mit dem Patientenrekrutierungsserver, beispielsweise über einen VPN-Tunnel. Insbesondere soll unter einem "Gesundheitsinformationssystem" ein Datenverarbeitungssystem einer Gesundheitsinstitution, insbesondere eines Krankenhauses, verstanden werden, das dazu vorgesehen ist, Patientendatensätze, insbesondere elektronische Patientenakten, für eine Verwendung in der und/oder durch die Gesundheitsinstitution zu verwalten. Vorzugsweise umfasst das Gesundheitsinformationssystem eine Client-Server-Struktur. Insbesondere ist ein Patientendatenserver des Gesundheitsinformationssystems dazu vorgesehen, die Patientendatensätze an eine der Patientendatenaufbereitungseinheiten insbesondere über ein Intranet des Gesundheitsinformationssystems zu versenden. Insbesondere soll unter einer "Suchroutine" eine Routine verstanden werden, die dazu vorgesehen ist, die Patientendatensätze zu finden, in denen zumindest ein vorgegebenes Rekrutierungsmerkmal vorkommt. Vorzugsweise ist die Suchroutine zumindest dazu vorgesehen, eine Rekrutierungsinformation an zumindest eine Einheit, insbesondere die Patientendatenaufbereitungseinheiten und/oder eine Indexdatenbank zumindest einer der Patientendatenaufbereitungseinheiten, zu übermitteln und ein Suchergebnis, insbesondere Angaben über eine Häufigkeit einer zutreffenden Suchanfrage des Patientenrekrutierungsservers, zu empfangen. Vorteilhaft enthält ein von den Patientendatenaufbereitungseinheiten übermitteltes Suchergebnis zumindest bei umfangreichen Anfragen, insbesondere mit mehreren Rekrutierungsmerkmalen, für jeden passenden Patienten und/oder Patientendatensatz jeweils einen Hinweis über zutreffende Suchkriterien und/oder Rekrutierungsmerkmale. Insbesondere ist der Patientenrekrutierungsserver dazu vorgesehen, anhand der Suchergebnisse ein aggregiertes Ergebnis und/oder einen aggregierten Report, insbesondere über mehrere Patientendatensätze, Suchläufe, Abfragen, Rekrutierungs-merkmale und/oder Sites, zu erzeugen und dieses/diesen insbesondere mittels einer Nutzerinterfaceroutine auszugeben. Unter "vorgesehen" soll insbesondere speziell programmiert, ausgelegt und/oder ausgestattet verstanden werden. Darunter, dass ein Objekt "zu einer bestimmten Funktion vorgesehen" ist, soll insbesondere verstanden werden, dass das Objekt diese bestimmte Funktion in zumindest einem Anwendungs- und/oder Betriebszustand erfüllt und/oder ausführt. Unter einem "Patientendatensatz" sollen Daten verstanden werden, die Informationen über einen in einer Gesundheitsinstitution, insbesondere einem Krankenhaus, zumindest untersuchten Patienten aufweisen. Die von den Patientendatenaufbereitungseinheiten empfangenen Patientendatensätze weisen Teile von elektronischen Patientenakten des Gesundheitsinformationssystems auf. Unter einem "Rekrutierungsmerkmal" soll insbesondere eine Vorgabe verstanden werden, die von einer Teilmenge der Patientendatensätze erfüllt wird. Das Rekrutierungsmerkmal kann Patientendatensätze in die Teilmenge aufnehmen oder aus der Teilmenge ausschließen. Das Rekrutierungsmerkmal ist als ein, dem Fachmann als sinnvoll erscheinendes Merkmal ausgebildet, beispielsweise als ein Mindestalter, als ein Höchstalter, als eine Diagnose, als ein Laborwert, als ein Sensorwert, als eine Medikamentierung und/oder als ein Gewicht. Insbesondere soll unter der Wendung "anhand von Rekrutierungsmerkmalen suchen" verstanden werden, dass die Suchroutine dazu vorgesehen ist, die Teilmenge der Patientendatensätze herauszufinden, die den Kriterien der Rekrutierungsmerkmale entspricht. Vorzugsweise ermittelt die Suchroutine zumindest Pseudonyminformationen der Patientendatensätze, die den Kriterien der Rekrutierungsmerkmale entsprechen.

Unter einer "Benachrichtigungsroutine" soll insbesondere eine Routine verstanden werden, die dazu vorgesehen ist, eine elektronische und/oder nicht-elektronische Nachricht, beispielsweise eine Liste, eine E-Mail, eine SMS und/oder eine Pager-Nachricht, zu versenden und/oder äquivalent ein Versenden durch eine dritte Stelle anzustoßen. Alternativ oder zusätzlich könnte die Benachrichtigungsroutine eine systeminterne Nachricht des Patientenrekrutierungssystems an das klinische Personal versenden. Unter einem "mittels der Rekrutierungsmerkmale gefundenen Patientenda tensatz" soll insbesondere ein Teil der durchsuchten Patientendatensätze verstanden werden, der den durch die Rekrutierungsmerkmale definierten Vorgaben entspricht. Insbesondere soll unter einer "Rekrutierungsnachricht" eine Nachricht verstanden werden, die das klinische Personal zumindest informiert, dass ein Patient rekrutiert werden soll. Vorzugsweise weist die Rekrutierungsnachricht die Rekrutierungsinformation zumindest eines Patientendatensatzes auf. Alternativ könnte die Rekrutierungsnachricht einen pseudonymisierten Patientendatensatz aufweisen.

Durch die erfindungsgemäße Ausgestaltung des Patientenrekrutierungssystems ist eine vorteilhaft zeitnahe Rekrutierung mit besonders geringem Personalaufwand möglich.

Des Weiteren wird vorgeschlagen, dass die Suchroutine dazu vorgesehen ist, die Patientendatensätze wiederholt anhand der Rekrutierungsmerkmale zu durchsuchen, wodurch neue und veränderte Patientendatensätze für die Rekrutierung vorteilhaft genutzt werden können. Alternativ oder zusätzlich könnte die Suchroutine nur neue und veränderte Patientendatensätze durchsuchen.

Ferner ist die erfindungsgemäße Benachrichtigungsroutine dazu vorgesehen, zu anhand der Rekrutierungsmerkmale neu gefundenen Patientendatensätzen automatisch die Rekrutierungsnachricht zu versenden, wodurch das klinische Personal vorteilhaft schnell über neue für die Rekrutierung geeignete Patienten informiert wird. Insbesondere sollen unter "neu gefundenen Patientendatensätzen" bei einem Suchdurchgang gefundene Patientendatensätze verstanden werden, die bei einem vorherigen Suchdurchgang nicht gefunden wurden. Insbesondere können die neu gefundenen Patientendatensätze zwischen den Suchdurchgängen neu zu den durchsuchten Patientendatensätzen hinzugekommen sein oder durch eine Veränderung der Rekrutierungsinformation und/oder der Rekrutierungsmerkmale erstmals gefunden worden sein. Unter "automatisch" soll insbesondere verstanden werden, dass die Benachrichtigungsroutine die Rekrutierungsnachricht ohne ein Zutun der Rekrutierungsstelle versendet. Alternativ könnte die Rekrutierungsnachricht nach einer Freigabe durch die Rekrutierungsstelle versendet werden.

Weiterhin sind die durchsuchten Patientendatensätze erfindungsgemäß als pseudonymisierte Patientendatensätze ausgebildet, wodurch die Patientendaten für eine Rekrutierung, insbesondere für eine klinische Studie, optimal genutzt werden können, ohne dass Datenschutzgrundsätze verletzt werden. Insbesondere soll unter einem "pseudonymisierten Patientendatensatz" ein Patientendatensatz verstanden werden, der keine Informationen aufweist, mittels deren ein Patient direkt und/oder einfach identifiziert werden kann.

Des Weiteren weist das erfindungsgemäße Patientenrekrutierungssystem mehrere verteilt angeordnete, insbesondere institutsinterne und/oder gesundheitsinstitutionsinterne, Patientendatenaufbereitungseinheiten auf, die in zumindest einem Betriebszustand jeweils Patientendatensätze von wenigstens einem Gesundheitsinformationssystem, insbesondere einem Krankenhausinformationssystem, empfangen, wodurch die Patientendatensätze innerhalb der Netzwerke der Gesundheitsinstitutionen vorteilhaft aufbereitet werden können. Insbesondere soll unter einer "Patientendatenaufbereitungseinheit" eine Einheit verstanden werden, die in zumindest einem Betriebszustand Patientendatensätze empfängt und diese aufbereitet. Vorzugsweise umfasst die Patientendatenaufbereitungseinheit zumindest einen Server, besonders bevorzugt mehrere Server. Insbesondere ist die Patientendatenaufbereitungseinheit an ein Gesundheitsinformationssystem einer Gesundheitsinstitution, insbesondere eines Krankenhauses, über eine Kommunikationsverbindung angeschlossen. Vorzugsweise ist die Patientendatenaufbereitungseinheit insbesondere durch eine IT-Infrastruktur der Gesundheitsinstitution, insbesondere des Krankenhauses, und/oder vorteilhaft durch in die Patientendatenaufbereitungseinheit integrierte Maßnahmen vor einem unberechtigten Zugriff geschützt. Erfindungsgemäß sind technische Vorkehrungen vorgesehen, die sicherstellen, dass auch die pseudonymisierten Patientendaten die Gesundheitsinstitution nicht verlassen können. Jede der Patientendatenaufbereitungseinheiten ist in einem lokalen Netz und/oder einem Intranet, insbesondere einer IT-Abteilung, eines Instituts und/oder einer Gesundheitsinstitution, insbesondere einem Krankenhaus, angeordnet. Patientendaten und/oder Patientendatensätze verlassen das lokale Netz und/oder das Intranet nicht. Hierdurch kann sichergestellt werden, dass dem Patientenrekrutierungssystem lediglich aggregierte Daten zur Verfügung gestellt werden, welche zur Darstellung von Suchergebnissen verwendbar sind. Vorzugsweise ist jede der Patientendatenaufbereitungseinheiten in ein Intranet unterschiedlicher Gesundheitsinstitutionen eingebunden.

Die erfindungsgemäßen Patientendatenaufbereitungseinheiten weisen jeweils zumindest eine Depseudonymisierungsroutine auf, die dazu vorgesehen sind, zumindest eine Patientenidentifikationsinformation zu einer Pseudonyminformation der pseudonymisierten Patientendatensätze zu bestimmen, wodurch eine Rekrutierung bei einem besonders wirksamen Datenschutz möglich ist. Unter einer "Depseudonymisierungsroutine" soll insbesondere eine Routine verstanden werden, die dazu vorgesehen ist, zu einer Pseudonyminformation eine dazugehörige Patientenidentifikationsinformation zu finden und insbesondere auszugeben. Vorzugsweise hat nur das klinische Personal Zugriff auf die von der
Depseudonymisierungsroutine bestimmte Patientenidentifikationsinformation. Insbesondere hat die Rekrutierungsstelle keinen Zugriff auf die von der Depseudonymisierungsroutine bestimmte Patientenidentifikationsinformation. Insbesondere soll unter einer "Patientenidentifikationsinformation" eine Information verstanden werden, die auf die Identität eines Patienten schließen lässt, beispielsweise ein Vorname, ein Nachname und ein Geburtsdatum. Alternativ oder zusätzlich könnte die Patientenidentifikationsinformation eine von dem Gesundheitsinformationssystem dem Patientendatensatz zugeordnete Identifikationsinformation aufweisen und zumindest der Name des Patienten mittels des Gesundheitsinformationssystems bestimmt werden. Unter einer "Pseudonyminformation" soll insbesondere eine Information verstanden werden, die eineindeutig einen Patientendatensatz identifiziert, ohne dass sie Rückschlüsse auf die Identität eines Patienten zulässt, beispielsweise eine Identifikationsnummer.

Des Weiteren umfassen die erfindungsgemäßen Patientendatenaufbereitungseinheiten jeweils zumindest einen Pseudonymverwaltungsserver, der in zumindest einem Betriebszustand die Pseudonyminformationen und die dazugehörigen Patientenidentifikationsinformationen speichert, wodurch die Patientenidentifikationsinformationen besonders effizient geschützt werden können. Unter einem "Pseudonymverwaltungsserver" soll insbesondere ein Server verstanden werden, der dazu vorgesehen ist, eine Zuordnung zwischen einer Pseudonyminformation und einer Patientenidentifikationsinformation zu ermöglichen. Vorzugsweise ist jedem Patientendatenaufbereitungsserver ein Pseudonymverwaltungsserver zugeordnet.

Ferner wird vorgeschlagen, dass die Rekrutierungsnachricht zumindest die Pseudonyminformationen und/oder die Patientenidentifikationsinformationen aufweist, wodurch der Patient, der rekrutiert werden soll, vorteilhaft identifiziert werden kann.

Weiterhin weisen die erfindungsgemäßen Patientendatenaufbereitungseinheiten jeweils zumindest eine Pseudonymisierungsroutine auf, die dazu vorgesehen sind, von Gesundheitsinformationssystemen empfangene Patientendatensätze zu pseudonymisieren, wodurch erreicht werden kann, dass nur Daten einen Einflussbereich der Gesundheitsinstitution verlassen, bei denen eine Zuordnung zu einem Patienten zumindest wesentlich erschwert ist. Unter einer "Pseudonymisierungsroutine" soll insbesondere eine Routine verstanden werden, die dazu vorgesehen ist, zumindest Informationen, die direkt auf eine Identität eines Patienten schließen lassen, wie beispielsweise Namen, Anschriften, Geburtsdaten und/oder Versicherungsnummern, aus dem Patientendatensatz zu entfernen. Vorzugsweise entfernt die Pseudonymisierungsroutine weitere, dem Fachmann als sinnvoll erscheinende Daten, die indirekt auf die Identität des Patienten schließen lassen und/oder die zur Patientenrekrutierung unnötig sind, aus dem Patientendatensatz. Vorzugsweise fügt die Pseudonymisierungsroutine dem Patientendatensatz eine eineindeutige Pseudonyminformation hinzu. Insbesondere kann die Pseudonymisierungsroutine zur Erzeugung der pseudonymisierten Patientendatensätze die Informationen der empfangenen Patientendatensätze kopieren und/oder verändern.

Des Weiteren sind die Patientendatenaufbereitungseinheiten erfindungsgemäß dazu vorgesehen, zumindest die Patientenidentifikationsinformation an klinisches Personal auszugeben, wodurch der zu einem Patientendatensatz gehörende Patient gegen unberechtigten Zugriff vorteilhaft geschützt von dem klinischen Personal ermittelt werden kann. Vorzugsweise sind die Pseudonymverwaltungsserver, besonders bevorzugt die Depseudonymisierungsroutinen der Pseudonymverwaltungsserver, dazu vorgesehen, zumindest die Patientenidentifikationsinformation an klinisches Personal auszugeben.Ferner wird vorgeschlagen, dass die Patientendatenaufbereitungseinheiten jeweils zumindest eine Nutzerinterfaceroutine aufweisen, die dazu vorgesehen sind, zumindest einen der Patientendatensätze zumindest teilweise an das klinische Personal auszugeben, wodurch dem klinischen Personal für die Rekrutierung vorteilhafte Informationen einfach und komfortabel zur Verfügung gestellt werden können. Vorzugsweise geben die Nutzerinterfaceroutinen den zumindest einen Patientendatensatz von der Patientenidentifikationsinformation und/oder von der Pseudonyminformation abhängig aus. Vorteilhaft geben die Nutzerinterfaceroutinen zumindest einen der pseudonymisierten Patientendatensätze und/oder zumindest einen der ursprünglichen Patientendatensätze aus. Insbesondere soll unter dem Begriff "jeweils" in diesem Zusammenhang verstanden werden, dass die Patientendatenaufbereitungseinheiten voneinander getrennt ausgebildete Nutzerinterfaceroutinen aufweisen. Vorzugsweise sind die Nutzerinterfaceroutinen jeweils nur aus einem Intranet des Gesundheitsinformationssystems und/oder nach einer Authentifizierung gegenüber dem Gesundheitsinformationssystem erreichbar. Insbesondere weisen die Nutzerinterfaceroutinen jeweils eine eigene Authentifizierungsroutine auf, mittels deren das berechtigte klinische Personal authentifiziert werden kann. Unter einer "Nutzerinterfaceroutine" soll insbesondere eine Routine verstanden werden, die dazu vorgesehen ist, Daten zur Darstellung aufzubereiten. Die Nutzerinterfaceroutine ist als eine, dem Fachmann als sinnvoll erscheinende Routine ausgebildet, vorzugsweise jedoch als ein http-Server. Vorzugsweise versendet die Nutzerinterfaceroutine Daten an ein zur Darstellung der Daten vorgesehenes Programm. Insbesondere ist die Nutzerinterfaceroutine dazu vorgesehen, die Daten lediglich nach einer erfolgreichen Authentifizierung, insbesondere Anwender-Authentifizierung, auszugeben. Insbesondere sollen unter einem "klinischen Personal" Personen verstanden werden, die in einer Gesundheitsinstitution, insbesondere einem Krankenhaus, für eine Betreuung und/oder eine Behandlung der Patienten zuständig sind.

Weiterhin wird vorgeschlagen, dass die Patientendatenaufbereitungseinheiten und insbesondere Nutzerinterfaceroutinen der Patientendatenaufbereitungseinheiten dazu vorgesehen sind, zumindest eine Patientenstatusinformation von dem klinischen Personal aufzunehmen, wodurch ein Ergebnis der Rekrutierung zeitnah und einfach ermittelt werden kann. Insbesondere soll unter einer "Patientenstatusinformation" eine Information verstanden werden, die einen Status eines Rekrutierungsverfahrens eines Patienten durch das klinische Personal beschreibt. Vorzugsweise bildet die Patientenstatusinformation dem Fachmann als sinnvoll erscheinende Status ab, besonders bevorzugt zumindest drei Status, und zwar vorteilhaft zumindest die Status "zur Rekrutierung vorgesehen", "ungeeignet" und "rekrutiert". Des Weiteren könnte die Patientenstatusinformation beispielsweise die Status "weitere Überprüfung notwendig" und "Rekrutierungsgespräch noch nicht erfolgt/möglich" abbilden.

Die Pseudonymverwaltungsserver versenden erfindungsgemäß in zumindest einem Betriebszustand die pseudonymisierten Patientendatensätze und die Patientendatenaufbereitungsserver der Patientendatenaufbereitungseinheiten empfangen erfindungsgemäß in zumindest einem Betriebszustand die pseudonymisierten Patientendatensätze von den Pseudonymverwaltungsservern, wodurch eine technische und räumliche Trennung zwischen dem Pseudonymverwaltungsserver und dem Patientendatenaufbereitungsserver erreicht werden kann und somit eine vorteilhaft hohe Datensicherheit möglich ist. Erfindungsgemäß kommunizieren die Pseudonymverwaltungsserver und die Patientendatenaufbereitungsserver nur über das Intranet des jeweiligen Gesundheitsinformationssystems, insbesondere verschlüsselt, beispielsweise über einen VPN-Tunnel. Vorzugsweise sind die Pseudonymverwaltungsserver und/oder die Patientendatenaufbereitungsserver als dedizierte Server ausgebildet, könnten aber alternativ auch als virtuelle Server oder als Server in einer Cloud, insbesondere des Gesundheitsinformationssystems, ausgebildet sein. Insbesondere ist der Pseudonymverwaltungsserver von dem Patientendatenaufbereitungsserver getrennt ausgebildet und über einen Kommunikationsweg, insbesondere über das Intranet des Gesundheitsinformationssystems, mit dem Patientendatenaufbereitungsserver verbunden. Alternativ könnte der Pseudonymverwaltungsserver in den Patientendatenaufbereitungsserver integriert sein. Vorzugsweise ist jedem Patientendatenaufbereitungsserver ein Pseudonymverwaltungsserver zugeordnet.

Ferner wird vorgeschlagen, dass die Patientendatenaufbereitungseinheiten jeweils zumindest eine Ontologieroutine aufweisen, die dazu vorgesehen ist, zumindest eine Ontologie der Patientendatensätze zu vereinheitlichen, wodurch auf unterschiedlichen Ontologien beruhende Patientendatensätze auf Grundlage einer Ontologie vorteilhaft durchsucht werden können. Vorzugsweise weist der Patientendatenaufbereitungsserver die Ontologieroutine auf. Alternativ oder insbesondere zusätzlich könnte der Patientenrekrutierungsserver eine Ontologieroutine aufweisen. Unter einer "Ontologie" soll dabei insbesondere eine insbesondere explizite formale Spezifikation einer Konzeptualisierung verstanden werden. Die Ontologie kann dabei insbesondere Listen, Thesauri, Instanzmodelle und/oder vorzugsweise Terminologien umfassen. Unter einer "Ontologieroutine" soll insbesondere eine Routine verstanden werden, die dazu vorgesehen ist, eine Bedeutung der Merkmale der Patientendatensätze zu analysieren und die Merkmale in Merkmale umzuwandeln, die der vereinheitlichten Terminologie entsprechen. Die Ontologieroutine ist als eine, dem Fachmann als sinnvoll erscheinende, insbesondere auf eine medizinische Terminologie optimierte Ontologieroutine ausgebildet, beispielsweise eine nach UMLS (Unified Medical Language System) und/oder nach der RxNorm arbeitende Routine. Vorzugsweise ist die Ontologieroutine dazu vorgesehen, die Merkmale der Patientendatensätze zumindest nach einer standardisierten Zielklassifikation der Krankheiten und/oder der Medizinprodukte oder ähnlicher Ontologien zu klassifizieren. Die Zielklassifikation der Krankheiten ist eine, dem Fachmann als sinnvoll erscheinende Zielklassifikation, vorteilhaft jedoch eine "ICD"-Klassifikation, beispielsweise ICD-10 oder zukünftige ICD- Versionen. Zielklassifikation der Medizinprodukte ist eine, dem Fachmann als sinnvoll erscheinende Klassifikation, vorteilhaft jedoch eine "ATC"-Klassifikation. Insbesondere erkennt die Ontologieroutine Merkmale, die nach einer von der Zielklassifikation verschiedenen Klassifikation, beispielsweise ICD-9, SNOMED oder NDC, klassifiziert sind, und wandelt diese Klassifikation in die Zielklassifikation um.

Vorzugsweise weist die Ontologieroutine eine Linguistikroutine auf. Vorzugsweise weist die Ontologieroutine eine Terminologisierungsroutine auf. Insbesondere ist die Linguistikroutine dazu vorgesehen, freien Text in die vereinheitlichte Ontologie, insbesondere eine vereinheitlichte Terminologie, zu überführen, wodurch auf dem freien Text beruhende Merkmale der vereinheitlichten Patientendatensätze vorteilhaft durchsucht werden können. Unter einer "Linguistikroutine" soll insbesondere eine Routine verstanden werden, die dazu vorgesehen ist, einen freien Text der Patientendatensätze zu analysieren und einzelnen Merkmalen des freien Texts eine Bedeutung zuzuordnen. Vorzugsweise ist die Linguistikroutine als eine, dem Fachmann als sinnvoll erscheinende Routine ausgebildet, besonders vorteilhaft als eine insbesondere auf medizinische Daten optimierte "GATE"-Routine (General Architecture for Text Engineering). Insbesondere soll unter einem "freien Text" ein Text verstanden werden, dessen Aufbau sich an Grammatikregeln der verwendeten Sprache orientiert. Insbesondere ist die Terminologisierungsroutine dazu vorgesehen, eine Terminologie der Patientendatensätze zumindest eines der Gesundheitsinformationssysteme in eine vereinheitlichte Terminologie zu überführen, wodurch die Patientendatensätze besonders effektiv durchsucht werden können. Unter einer "Terminologisierungsroutine" soll insbesondere eine Routine verstanden werden, die dazu vorgesehen ist, unterschiedliche Terminologien der Patientendatensätze zu analysieren, einzelnen Merkmalen der Patientendatensätze eine Bedeutung zuzuordnen und die Merkmale anhand der Bedeutung in eine andere, vereinheitlichte Terminologie zu übersetzen.

Des Weiteren wird vorgeschlagen, dass die Patientendatenaufbereitungseinheiten jeweils zumindest eine Datenbank aufweisen, die dazu vorgesehen ist, die pseudonymisierten Patientendatensätze zu speichern, wodurch auf die Patientendatensätze vorteilhaft schnell zugegriffen werden kann. Vorzugsweise weisen die Patientendatenaufbereitungsserver der Patientendatenaufbereitungseinheiten jeweils eine Datenbank auf. Unter einer "Datenbank" soll insbesondere ein Mittel des Patientenrekrutierungssystems verstanden werden, das dazu vorgesehen ist, die Patientendatensätze zu speichern. Vorzugsweise weist die Datenbank mehrere Tabellen auf. Die Datenbank ist als eine, dem Fachmann als sinnvoll erscheinende Datenbank ausgebildet, beispielsweise als eine SQL-Datenbank

Zudem wird vorgeschlagen, dass der Patientenrekrutierungsserver zumindest eine Nutzerinterfaceroutine aufweist, die dazu vorgesehen ist, zumindest ein Suchergebnis der Suchroutine an eine Rekrutierungsstelle auszugeben, wodurch eine Auswahl der Rekrutierungsmerkmale vorteilhaft überprüft werden kann. Insbesondere sollen unter einem "Suchergebnis" Informationen verstanden werden, die dazu vorgesehen sind, dem Nutzer ein Ergebnis seiner Recherche zu vermitteln. Vorzugsweise umfasst das Suchergebnis zumindest die Pseudonyminformationen und/oder eine Patientenidentifikationsinformation der gefundenen Patientendatensätze. Bevorzugt weist das Suchergebnis mindestens eine Information über eine Anzahl der gefundenen Patientendatensätze auf. Vorteilhaft umfasst das Suchergebnis die gefundenen Patientendatensätze. Alternativ oder zusätzlich könnte das Suchergebnis Verweise auf die gefundenen Patientendatensätze enthalten. Besonders vorteilhaft umfasst das Suchergebnis lediglich Daten, die die gefundenen Patientendatensätze beschreiben, wodurch ein besonders guter Datenschutz erreicht werden kann. Unter einer "Rekrutierungsstelle" soll insbesondere ein Nutzer des Patientenrekrutierungssystems verstanden werden, der mittels des Patientenrekrutierungssystems Patienten, insbesondere für eine klinische Studie, finden will. Insbesondere soll unter "ausgeben" verstanden werden, dass die Rekrutierungsstelle das Suchergebnis an eine Vorrichtung sendet, die das Suchergebnis dem Nutzer darstellt.

In einer vorteilhaften Ausbildung der Erfindung wird vorgeschlagen, dass die Nutzerinterfaceroutine des Patientenrekrutierungsservers dazu vorgesehen ist, eine Rekrutierungsinformation von der Rekrutierungsstelle aufzunehmen, wodurch die Rekrutierungsstelle insbesondere konstruktiv die Patientendatensätze nach unterschiedlichen Rekrutierungsmerkmalen suchen kann. Insbesondere soll unter einer "Rekrutierungsinformation" eine Information verstanden werden, die angibt, dass ein Patient zur Rekrutierung vorgesehen ist. Vorzugsweise wählt die Rekrutierungsstelle in dem Suchergebnis die Patienten aus, die die Rekrutierungsstelle, insbesondere für eine klinische Studie, rekrutieren will. Vorzugsweise weist die Rekrutierungsinformation zumindest die Pseudonyminformationen der anhand ihrer Patientendatensätze ausgewählten Patienten auf. Alternativ könnte die Rekrutierungsinformation die entsprechenden Pseudonyminformationen markieren.

In einer weiteren Ausgestaltung wird vorgeschlagen, dass die Patientendatenaufbereitungseinheiten dazu vorgesehen sind, die pseudonymisierten Patientendatensätze anhand von durch die Rekrutierungsinformation verknüpften Rekrutierungsmerkmalen zu suchen, wodurch komplexe Durchsuchungen insbesondere konstruktiv einfach möglich sind. Eine Verknüpfung der Rekrutierungsmerkmale durch die Rekrutierungsinformation kann dabei durch wahrscheinlichkeitsbasierte Algorithmen oder ähnliche Algorithmen des maschinellen Lernens und/oder durch eine Fuzzylogik und/oder vorzugsweise durch logische Operationen, wie insbesondere UND, ODER, NICHT, KLEINER, GRÖSSER und/oder Klammern, die die Rekrutierungsmerkmale miteinander in Beziehung setzen, erfolgen.

Des Weiteren wird vorgeschlagen, dass die Nutzerinterfaceroutine des Patientenrekrutierungsservers dazu vorgesehen ist, eine Anzahl von gefundenen Patientendatensätzen für einzelne der verknüpften Rekrutierungsmerkmale auszugeben, wodurch vorteilhaft schnell erkannt werden kann, welche Rekrutierungsmerkmale die Anzahl der gefundenen Patientendatensätze wie stark einschränken. Insbesondere soll unter "einer Anzahl von gefundenen Patientendatensätzen" eine Information verstanden werden, die angibt, wie viele Patientendatensätze, hier für jedes der Rekrutierungsmerkmale einzeln, gefunden worden sind.

Ferner wird vorgeschlagen, dass die Patientendatenaufbereitungseinheiten jeweils zumindest eine Indexroutine aufweisen, die dazu vorgesehen sind, die Patientendatensätze zu indizieren und/oder zu einer, insbesondere semantischen, Suche aufzubereiten, wodurch eine besonders schnelle Suche in den Patientendatensätzen erreicht werden kann. Unter einer "Indexroutine" soll in diesem Zusammenhang insbesondere eine Routine verstanden werden, die dazu vorgesehen ist, eine für eine Durchsuchung optimierte Information zu den Patientendatensätzen zu erzeugen. Ferner ist die Indexroutine vorteilhaft dazu vorgesehen, Statistiken, wie beispielsweise über eine Häufigkeit eines Rekrutierungsmerkmals, insbesondere von vorkommenden Diagnosen, Laborwerten, Behandlungen und/oder Prozeduren, zu erstellen. Vorzugsweise verwendet die Indexroutine die von der Terminologisierungsroutine den Merkmalen der Patientendatensätze zugeordneten Bedeutungen.

Ferner wird vorgeschlagen, dass die Nutzerinterfaceroutine des Patientenrekrutierungsservers dazu vorgesehen ist, eine Anzahl von gefundenen Patientendatensätzen, die von einem der Gesundheitsinformationssysteme stammen, auszugeben, wodurch Gesundheitsinstitutionen, insbesondere Krankenhäuser, mit wenigen geeigneten Patienten vorteilhaft erkannt werden können. Unter "die von einem der Gesundheitsinformationssysteme stammen" soll insbesondere verstanden werden, dass die Nutzerinterfaceroutine angibt, wie viele Patienten aus jedem der Gesundheitsinstitutionen, die die Gesundheitsinformationssysteme betreiben, stammen.

In einer vorteilhaften Ausbildung der Erfindung wird vorgeschlagen, dass die Patientendatensätze biomedizinische Daten, Medizinproduktdaten, Labordaten, Personendaten und/oder Sensordaten aufweisen. wodurch eine besonders exakte Auswahl der Patienten möglich ist. Unter "biomedizinischen Daten" sollen insbesondere dem Fachmann als sinnvoll erscheinende Daten verstanden werden, die eine Diagnose und/oder eine Untersuchung durch das klinische Personal betreffen, und zwar vorteilhaft zumindest Symptome, Diagnosen, Prozeduren, Behandlungen, Therapiedaten und/oder genetische Daten. Insbesondere sollen unter "Medizinproduktdaten" Daten verstanden werden, die beschreiben, mit welchen Medizinprodukten ein Patient behandelt worden ist, behandelt wird und/oder behandelt werden soll, wie insbesondere medizinische Behandlungsgeräte, beispielsweise eine Behandlung mit einer Beatmungsmaschine, und/oder vorteilhaft Medikamente. Unter "Labordaten" sollen insbesondere Daten verstanden werden, die durch ein medizinisches Labor ermittelt worden sind. Insbesondere sollen unter "Personendaten" nichtmedizinische Daten verstanden werden, die den Patienten beschreiben, beispielsweise eine Körpergröße, ein Alter, ein Geschlecht, ein Gewicht und/oder andere, dem Fachmann als sinnvoll erscheinende Daten. Unter "Sensordaten" sollen insbesondere Daten verstanden werden, die ein Sensor von dem Patienten aufnimmt, beispielsweise ein Elektrokardiogramm, ein Blutdruck, ein Puls, ein Blutzuckerwert und/oder andere, dem Fachmann als sinnvoll erscheinende Daten.

Des Weiteren geht die Erfindung aus von einem Patientenrekrutierungsverfahren, insbesondere mit einem erfindungsgemäßen Patientenrekrutierungssystem, wobei Patientendatensätze anhand von Rekrutierungsmerkmalen durchsucht werden und eine Rekrutierungsnachricht versandt wird, wenn mittels der Rekrutierungsmerkmale zumindest ein Patientendatensatz gefunden wird.

Das erfindungsgemäße Patientenrekrutierungssystem und das erfindungsgemäße Patientenrekrutierungsverfahren sollen hierbei nicht auf die oben beschriebene Anwendung und Ausführungsform beschränkt sein. Insbesondere können das erfindungsgemäße Patientenrekrutierungssystem und das erfindungsgemäße Patientenrekrutierungsverfahren zu einer Erfüllung einer hierin beschriebenen Funktionsweise eine von einer hierin genannten Anzahl von einzelnen Elementen, Bauteilen und Einheiten abweichende Anzahl aufweisen.

### Zeichnung

Weitere Vorteile ergeben sich aus der folgenden Zeichnungsbeschreibung. In der Zeichnung ist ein Ausführungsbeispiel der Erfindung dargestellt. Die Zeichnung, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: ein erfindungsgemäßes Patientenrekrutierungssystem zur Durchführung eines erfindungsgemäßen Patientenrekrutierungsverfahrens in einer schematischen Darstellung,
- Fig. 2: eine Pseudonymisierung von Patientendatensätzen mittels des Patientenrekrutierungsverfahrens aus Figur 1,
- Fig. 3: eine Durchsuchung von Patientendatensätzen mittels des Patientenrekrutierungsverfahrens aus Figur 1,
- Fig. 4: eine Benachrichtigung von klinischem Personal mittels des Patientenrekrutierungsverfahrens aus Figur 1 und
- Fig. 5: eine Aufnahme einer Rekrutierungsinformation von dem klinischen Personal mittels des Patientenrekrutierungsverfahrens aus Figur 1.

### Beschreibung des Ausführungsbeispiels

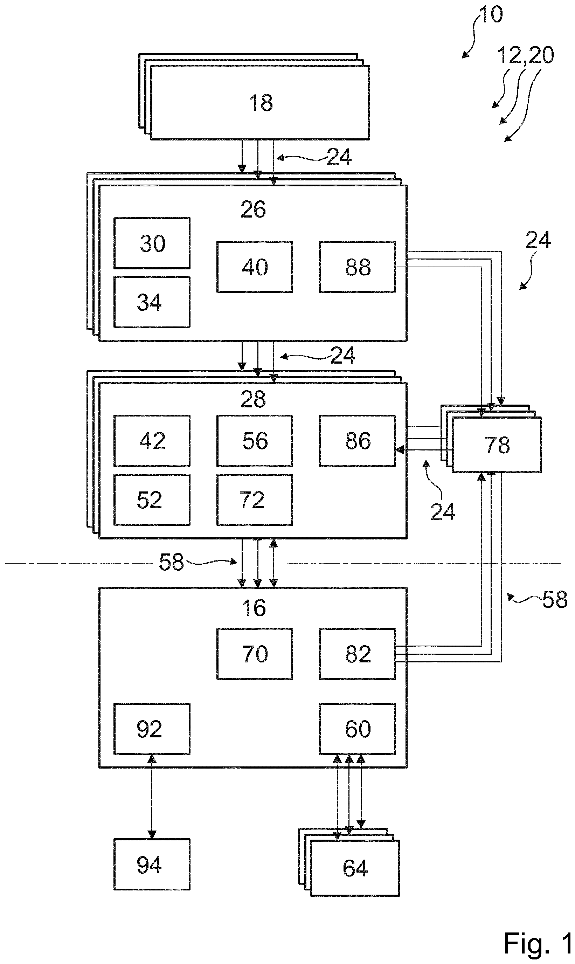

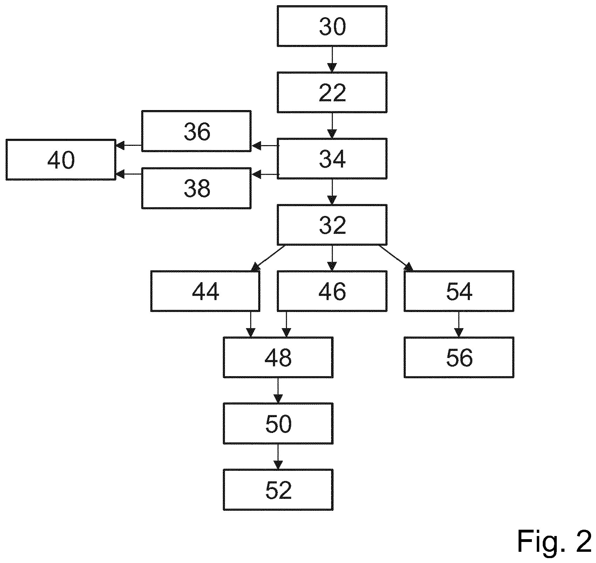

Figur 1 zeigt ein erfindungsgemäßes Patientenrekrutierungssystem 10 zur Durchführung eines erfindungsgemäßen Patientenrekrutierungsverfahrens, das in den Figuren 2 bis 5 näher beschrieben ist. Das Patientenrekrutierungssystem 10 weist mehrere in unterschiedlichen Krankenhäusern 12 angeordnete Patientendatenaufbereitungseinheiten 14 und zumindest einen Patientenrekrutierungsserver 16 auf. In den Figuren 2 bis 5 ist eine Rekrutierung von Patienten mittels einer der Patientendatenaufbereitungseinheiten 14 beschrieben. Eine Rekrutierung mittels einer anderen der Patientendatenaufbereitungseinheiten 14 erfolgt äquivalent.

Die Patientendatenaufbereitungseinheiten 14 empfangen in zumindest einem Betriebszustand von Patientendatenservern 18 eines Gesundheitsinformationssystems 20 der jeweiligen Krankenhäuser 12 ursprüngliche Patientendatensätze 22. Die Patientendatenserver 18 senden den Patientendatenaufbereitungseinheiten 14 selbstständig neu angelegte und veränderte Patientendatensätze 22. Die Patientendatenaufbereitungseinheiten 14 und die Patientendatenserver 18 sind über ein Intranet 24 des Gesundheitsinformationssystems 20 miteinander verbunden. Die Patientendatensätze 22 sind als elektronische Gesundheitsakten ausgebildet. Die Patientendatensätze 22 weisen biomedizinische Daten, Medizinproduktdaten, Labordaten, Personendaten und/oder Sensordaten auf.

Die Patientendatenaufbereitungseinheiten 14 weisen jeweils einen Pseudonymverwaltungsserver 26 und einen Patientendatenaufbereitungsserver 28 auf. Transferroutinen 30 der Pseudonymverwaltungsserver 26 empfangen in zumindest einem Betriebszustand die Patientendatensätze 22 von den jeweiligen Patientendatenservern 18. Die Pseudonymverwaltungsserver 26 sind dazu vorgesehen, die Patientendatensätze 22 zu pseudonymisieren und pseudonymisierte Patientendatensätze 32 zu erzeugen. Die Pseudonymverwaltungsserver 26 leiten die pseudonymisierten Patientendatensätze 32 an die Patientendatenaufbereitungsserver 28 weiter.

Figur 2 zeigt einen Teil des Patientenrekrutierungsverfahrens, der die Patientendatensätze 22 der Patientendatenserver 18 pseudonymisiert und eine Terminologie der Patientendatensätze 22 zur Indizierung vereinheitlicht. Die Transferroutinen 30 der Pseudonymverwaltungsserver 26 fordern von den Patientendatenservern 18 in vorgegebenen Intervallen zumindest veränderte oder neue der ursprünglichen Patientendatensätze 22 von den Patientendatenservern 18 an.

Pseudonymisierungsroutinen 34 der Pseudonymverwaltungsserver 26 sind jeweils dazu vorgesehen, die empfangenen ursprünglichen Patientendatensätze 22 zu pseudonymisieren und die pseudonymisierten Patientendatensätze 32 zu erzeugen. Dazu durchsucht die Pseudonymisierungsroutine 34 jeweils den gesamten empfangenen ursprünglichen Patientendatensatz 22 nach Informationen, die den Patienten, zu dem der Patientendatensatz 22 gehört, direkt identifizieren können. Die Pseudonymisierungsroutine 34 erzeugt den pseudonymisierten Patientendatensatz 32, der diese Informationen nicht enthält. Die Pseudonymisierungsroutine 34 weist selbstlernende Funktionen auf, um die Informationen, die den Patienten direkt identifizieren können, aus dem ggf. vorhandenen freien Text zu entfernen.

Die Pseudonymisierungsroutine 34 erzeugt eine Pseudonyminformation 36, die den pseudonymisierten Patientendatensatz 32 eineindeutig identifiziert, und fügt diese dem pseudonymisierten Patientendatensatz 32 hinzu. Die Pseudonymisierungsroutine 34 bestimmt aus Informationen, die den Patienten, zu dem der ursprüngliche Patientendatensatz 22 gehört, direkt identifizieren können, eine Patientenidentifikationsinformation 38. Die Patientenidentifikationsinformation 38 weist zumindest einen Namen, einen Vornamen und vorteilhaft ein Geburtsdatum des Patienten auf. Somit ist es möglich, mittels der Patientenidentifikationsinformation 38 den Patienten eineindeutig zu identifizieren.

Die Pseudonymisierungsroutine 34 speichert die Pseudonyminformation 36 und die Patientenidentifikationsinformation 38, einander zugeordnet, an eine Pseudonymdatenbank 40 des jeweiligen Pseudonymverwaltungsservers. 26Die Pseudonymdatenbank 40 speichert die Pseudonyminformation 36 und die Patientenidentifikationsinformation 38 einander zugeordnet. Die Pseudonymdatenbank 40 ist speziell gegen unberechtigte Zugriffe geschützt.

Der Pseudonymverwaltungsserver 26 sendet den pseudonymisierten Patientendatensatz 32 an den Patientendatenaufbereitungsserver 28. Der Pseudonymverwaltungsserver 26 und der Patientendatenaufbereitungsserver 28 kommunizieren nur über das Intranet 24 des Gesundheitsinformationssystems 20 miteinander. Der Patientendatenaufbereitungsserver 28 weist eine Ontologieroutine 42 auf.

Die Ontologieroutine 42 ist dazu vorgesehen, zumindest eine Ontologie der pseudonymisierten Patientendatensätze 32 zur Indizierung zu vereinheitlichen. Alternativ oder zusätzlich könnte die Ontologieroutine 42 einen vereinheitlichten Patientendatensatz erzeugen. Die Ontologieroutine 42 umfasst eine Linguistikroutine 44 und eine Terminologisierungsroutine 46. Die Linguistikroutine 44 ist dazu vorgesehen, die Bedeutung von Merkmalen von freiem Text der Patientendatensätze 32 zu analysieren. Dazu weist die Linguistikroutine 44 eine "GATE"-Instanz auf. Die Linguistikroutine 44 ermittelt aus dem freien Text Merkmale, die nach einem System, insbesondere ICD-10, kodiert sind.

Die Terminologisierungsroutine 46 ist dazu vorgesehen, eine Terminologie der Merkmale der Patientendatensätze 32 in eine vereinheitlichte Terminologie zu überführen. Die ursprünglichen Patientendatensätze 22 der verschiedenen Gesundheitsinformationssysteme 20 können nach unterschiedlichen Systemen kodierte Merkmale aufweisen. Die Terminologisierungsroutine 46 weist Instanzen von dem Fachmann als sinnvoll erscheinenden Überführungsbibliotheken auf, die die unterschiedlich kodierten Merkmale in gleich kodierte Merkmale übersetzen. Hier umfasst die Terminologisierungsroutine 46 zumindest eine UMLS-Instanz und eine RxNorm-Instanz.

Des Weiteren ist die Ontologieroutine 42 dazu vorgesehen, zu den Patientendatensätzen 32 weitere, dem Fachmann als sinnvoll erscheinende Merkmalen zu ermitteln, beispielsweise Informationen über das Krankenhaus 12 und/oder Qualitätsinformationen insbesondere der Terminologisierung. Zudem könnten zur Ermittlung der Merkmale selbstlernende Funktionen eingesetzt werden.

Der Patientendatenaufbereitungsserver 28 weist eine Indexroutine 48 auf, die dazu vorgesehen ist, die von der Ontologieroutine 42 ermittelten Merkmale zu indizieren. Die Indexroutine 48 speichert eine Indexinformation 50 mit den indizierten Merkmalen in einer Indexdatenbank 52 des Patientendatenaufbereitungsservers 28.

Der Patientendatenaufbereitungsserver 28 weist eine Datenbankroutine 54 auf, die dazu vorgesehen ist, die pseudonymisierten Patientendatensätze 32 zu speichern. Die Datenbankroutine 54 speichert die pseudonymisierten Patientendatensätze 32 in einer Datenbank 56 des Patientendatenaufbereitungsservers 28. Die Indexinformationen 50 weisen jeweils einen Verweis auf einen der pseudonymisierten Patientendatensätze 32 in der Datenbank 56 auf.

Der Patientenrekrutierungsserver 16 ist an einem anderen Ort angeordnet als die Patientendatenaufbereitungseinheiten 14. Hier sind die Patientendatenaufbereitungseinheiten 14 und der Patientenrekrutierungsserver 16 über eine verschlüsselte Verbindung über das Internet 58 miteinander verbunden, und zwar über einen VPN-Tunnel.

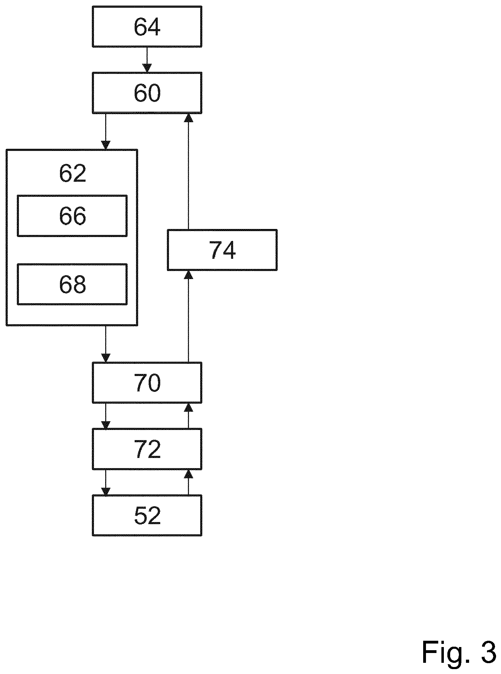

Wie Figur 3 zeigt, weist der Patientenrekrutierungsserver 16 eine Nutzerinterfaceroutine 60 auf, die dazu vorgesehen ist, Informationen Nutzern des Patientenrekrutierungssystems 10 bereitzustellen und von den Nutzern aufzunehmen. Hier umfasst die Nutzerinterfaceroutine 60 einen HTML-Server. Alternativ sind jedoch auch beliebige andere Servertypen denkbar. Ferner könnte die Nutzerinterfaceroutine 60 als ein Server einer Server-Client-Struktur ausgebildet sein, wobei auf Computern der Nutzer installierte und ausgeführte Programme des Patientenrekrutierungssystems 10 die Informationen an die Nutzer ausgeben und Informationen von den Nutzern aufnehmen.

Die Nutzerinterfaceroutine 60 des Patientenrekrutierungsservers 16 ist dazu vorgesehen, eine Rekrutierungsinformation 62 von dem Nutzer aufzunehmen, der eine Rekrutierungsstelle 64 bildet. Die Rekrutierungsstelle 64 besteht beispielsweise aus Vertretern eines Pharmazieunternehmens, das eine klinische Studie durchführen will, aus Ärzten, die die klinische Studie durchführen und ggf. aus Experten in der Bedienung des Patientenrekrutierungssystems 10. Für die klinische Studie sucht die Rekrutierungsstelle 64 geeignete Patienten, die sie zur Durchführung der Studie rekrutieren möchte.

Die Rekrutierungsinformation 62 weist Rekrutierungsmerkmale 66 und Operatoren 68 auf. Die Operatoren 68 verknüpfen die Rekrutierungsmerkmale 66 logisch miteinander. Durch die Operatoren 68 verknüpfte Rekrutierungsmerkmale 66 können, beispielsweise durch Klammern, gruppiert werden. Es kann somit aus den Rekrutierungsmerkmalen 66 ein beliebiger logischer Ausdruck gebildet werden. Die Eingabe der Rekrutierungsinformation 62 erfolgt textbasiert und wird mittels eines Formelanalysators geprüft und umgesetzt. Alternativ oder zusätzlich könnte eine Eingabe der Rekrutierungsinformation 62 auch graphisch erfolgen.

Die Rekrutierungsmerkmale 66 können biomedizinische Daten, Medizinproduktdaten, Labordaten, Personendaten und/oder Sensordaten aufweisen. Die Rekrutierungsmerkmale 66 sind zumindest aus einer Menge von kodierten Merkmalen selektierbar, die bei der Vereinheitlichung der Patientendatensätze 32 zur Verfügung stehen. Zudem können durch die Rekrutierungsmerkmale 66 und die Operatoren 68 Wertebereiche festgelegt werden. Des Weiteren können die Rekrutierungsmerkmale 66 freie Suchbegriffe aufweisen, nach denen der freie Text der Patientendatensätze 22 durchsucht wird.

Der Patientenrekrutierungsserver 16 weist eine Suchroutine 70 auf, die dazu vorgesehen ist, pseudonymisierte Patientendatensätze 32 mittels der Indexinformationen 50 der Indexdatenbank 52 der Patientendatenaufbereitungseinheiten 14 anhand der Rekrutierungsinformation 62 zu durchsuchen. Die Suchroutine 70 des Patientenrekrutierungsservers 16 sendet zumindest die Rekrutierungsinformation 62 an Suchroutinen 72 der Patientendatenaufbereitungseinheiten 14. Die Suchroutinen 72 der Patientendatenaufbereitungseinheiten 14 durchsuchen die Indexdatenbanken 52 der Patientendatenaufbereitungseinheiten 14 anhand der Rekrutierungsinformation 62. An welche der Patientendatenaufbereitungseinheiten 14 die Rekrutierungsinformationen 62 gesendet werden und welche der Patientendatenaufbereitungseinheiten 14 mittels der Rekrutierungsinformation 62 eine Suche durchführt, ist einstellbar. Die Suchroutine 72 erstellt ein Suchergebnis 74 und sendet das Suchergebnis 74 an den Patientenrekrutierungsserver 16.

Das Suchergebnis 74 weist zumindest eine Information über eine Anzahl der gefundenen Patientendatensätze 32 auf. Zudem weist das Suchergebnis 74 eine Anzahl von gefundenen Patientendatensätzen 32 für jedes der Rekrutierungsmerkmale 66 aufgeschlüsselt und insbesondere für gruppierte Rekrutierungsmerkmale 66 aufgeschlüsselt auf. Zudem weist das Suchergebnis 74 eine Anzahl von gefundenen Patientendatensätzen 32 für jedes der Krankenhäuser 12 aufgeschlüsselt auf. Des Weiteren könnte das Suchergebnis 74 weitere, dem Fachmann als sinnvoll erscheinende Informationen über die gefundenen Patientendatensätze 32 und/oder zumindest Teile der gefundenen Patientendatensätze 32 aufweisen. Zudem weist das Suchergebnis 74 die Pseudonyminformation 36 der gefundenen Patientendatensätze 32 auf. Insbesondere weist das Suchergebnis 74 keine Informationen auf, die dazu geeignet sind, einen Patienten, dem einer der gefundenen Patientendatensätzen 32 zugeordnet ist, direkt zu identifizieren.

Die Nutzerinterfaceroutine 60 ist dazu vorgesehen, das Suchergebnis 74 der Suchroutine 70 an die Rekrutierungsstelle 64 auszugeben. Dabei gibt die Nutzerinterfaceroutine 60 die Anzahl von gefundenen Patientendatensätzen 32 für einzelne der logisch verknüpften Rekrutierungsmerkmale 66 und für eine Anzahl von gefundenen Patientendatensätzen 32, die von einem der Gesundheitsinformationssysteme 20 stammen, aus.

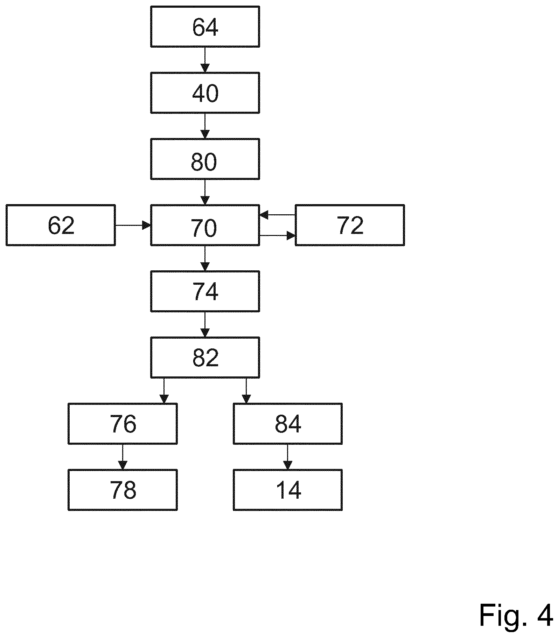

Wie Figur 4 zeigt, ist der Patientenrekrutierungsserver 16 dazu vorgesehen, eine Rekrutierungsnachricht 76 an klinisches Personal 78 der Krankenhäuser 12 zu senden. Die Nutzerinterfaceroutine 60 ist dazu vorgesehen, eine Rekrutierungsaktivierungsinformation 80 von der Rekrutierungsstelle 64 aufzunehmen. Die Rekrutierungsaktivierungsinformation 80 aktiviert eine Rekrutierung für eine neue oder vorteilhaft eine von den Suchroutinen 72 der Patientendatenaufbereitungseinheiten 14 gespeicherte Rekrutierungsinformation 62. Die Suchroutinen 72 der Patientendatenaufbereitungseinheiten 14 durchsuchen anhand der Rekrutierungsinformation 62 die Indexdatenbank 52 nach entsprechenden Patientendatensätzen 32. Die Suchergebnisse 74 der Suche gibt die Suchroutine 72 an eine Benachrichtigungsroutine 82 des Patientenrekrutierungsservers 16 weiter. Das Suchergebnis 74 weist hierbei eine Information auf, die beschreibt, wie das klinische Personal 78 kontaktierbar ist.

Die Suchroutinen 72 der der Patientendatenaufbereitungseinheiten 14 führen die Suche mittels der Rekrutierungsinformation 62 wiederholt durch, und zwar hier in einem von der Rekrutierungsstelle 64 bestimmbaren Intervall. Alternativ könnte die Suchroutine 72 nur neue und veränderte Patientendatensätze 32 durchsuchen.

Die Benachrichtigungsroutine 82 versendet zu den in dem Suchergebnis 74 aufgeführten Patientendatensätzen 32 eine Rekrutierungsnachricht 76 an das klinische Personal 78 des entsprechenden Krankenhauses 12, und zwar hier als Push-Nachricht. Der Patientenrekrutierungsserver 16 sendet eine Rekrutierungsnachricht 76 an ein Empfangsgerät, das einer Person des klinischen Personals 78 persönlich zugeordnet ist, hier an einen Pager und/oder ein Mobiltelefon. Die Rekrutierungsnachricht 76 gibt zumindest an, dass anhand der Rekrutierungsinformation 62 ein Patientendatensatz 32 zu einem in dem Krankenhaus 12 behandelten Patienten gefunden wurde. Die Rekrutierungsnachricht 76 könnte dem Fachmann als sinnvoll erscheinende Informationen aufweisen, beispielsweise die Pseudonyminformation 36 und/oder eine Anzahl von gefundenen Patientendatensätzen 32. Die Benachrichtigungsroutine 82 ist dazu vorgesehen, zu den anhand der Rekrutierungsinformation 62 neu gefundenen Patientendatensätzen 32 automatisch Rekrutierungsnachrichten 76 an das klinische Personal 78 zu versenden. Alternativ zu dem Patientenrekrutierungsserver 16 könnte die Patientendatenaufbereitungseinheit 14 die Benachrichtigungsroutine 82 aufweisen.

Zudem ist die Benachrichtigungsroutine 82 dazu vorgesehen, eine Rekrutierungsbeschreibungsinformation 84 an die Patientendatenaufbereitungseinheit 14 zu senden. Die Rekrutierungsbeschreibungsinformation 84 enthält Angaben über ein Verfahren, für das der Patient rekrutiert werden soll, insbesondere über Risiken des Verfahrens und/oder über notwendige Voraussetzungen zur Teilnahmen an dem Verfahren.

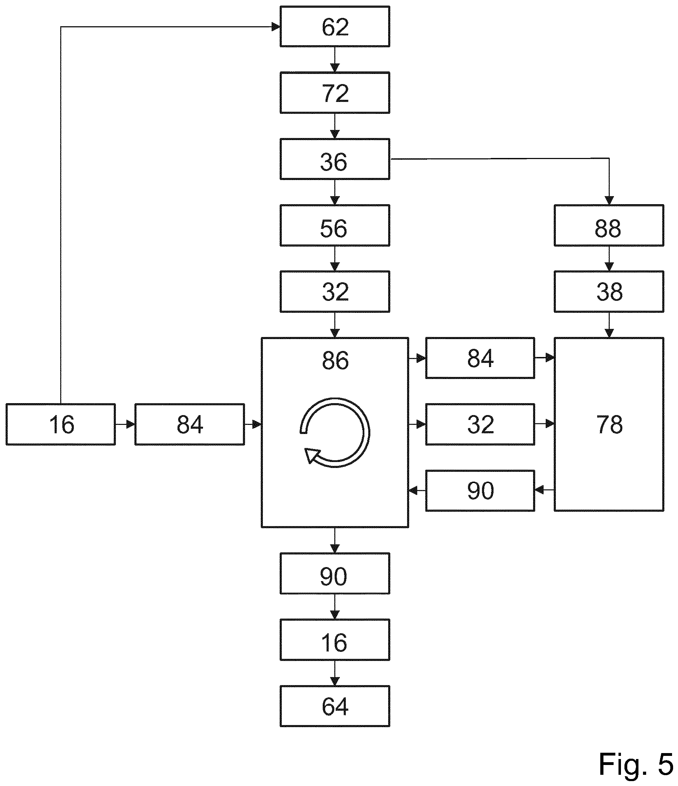

Wie Figur 5 zeigt, empfängt die Patientendatenaufbereitungseinheit 14 die Rekrutierungsinformation 62 Die Suchroutine 72 der Patientendatenaufbereitungseinheit 14 ermittelt zu der Rekrutierungsinformation 62 die Pseudonyminformationen 36. Wie zu Figur 4 beschrieben versendet der Patientenrekrutierungsserver 16 die Rekrutierungsnachricht 76 an das klinische Personal 78. Die Datenbank 56 ermittelt die zu den Pseudonyminformationen 36 gehörenden Patientendatensätze 32 und stellt sie einer Nutzerinterfaceroutine 86 der Patientendatenaufbereitungseinheit 14, hier des Patientendatenaufbereitungsservers 28, zur Verfügung.

Der Pseudonymverwaltungsserver 26 weist eine Depseudonymisierungsroutine 88 auf, die dazu vorgesehen ist, zumindest die Patientenidentifikationsinformationen 38 zu den Pseudonyminformationen 36 der pseudonymisierten Patientendatensätze 32 zu bestimmen. Die Depseudonymisierungsroutine 88 fragt bei der Pseudonymdatenbank 40 die zu den Pseudonyminformationen 36 gehörenden Patientenidentifikationsinformationen 38 ab. Die Depseudonymisierungsroutine 88 umfasst ein Interface, das dazu vorgesehen ist, die Patientenidentifikationsinformationen 38 dem klinischen Personal 78 auszugeben. Hier ist das Interface der Depseudonymisierungsroutine 88 als ein http-Server ausgebildet. Das Interface ist gegen unberechtigten Zugriff geschützt.

Die Nutzerinterfaceroutine 86 des Patientendatenaufbereitungsservers 28 ist dazu vorgesehen, die Patientenidentifikationsinformationen 38 an das klinische Personal 78 auszugeben. Zudem ist die Nutzerinterfaceroutine 86 dazu vorgesehen, die pseudonymisierten Patientendatensätze 32 an das klinische Personal 78 auszugeben. Ferner könnte die Nutzerinterfaceroutine 86 dazu vorgesehen sein, die ursprünglichen Patientendatensätze 22 an das klinische Personal 78 auszugeben. Des Weiteren ist die Nutzerinterfaceroutine 86 dazu vorgesehen, die Rekrutierungsbeschreibungsinformationen 84 an das klinische Personal 78 auszugeben. Durch diese Informationen ist das klinische Personal 78 in der Lage, die Rekrutierung zu überprüfen und ein Rekrutierungsgespräch zu führen.

Die Nutzerinterfaceroutine 86 ist als ein http-Server ausgebildet. Alternativ sind jedoch auch beliebige andere Servertypen denkbar. Das klinische Personal 78 ruft eine von der Nutzerinterfaceroutine 86 bereitgestellte Webseite auf. Die Webseite weist eine Funktion zur Authentifizierung des klinischen Personals 78 auf. Die Webseite ruft von einem Computer des klinischen Personals 78 aus die Patientenidentifikationsinformationen 38 von der Depseudonymisierungsroutine 88 des Pseudonymverwaltungsservers 26 ab.

Das klinische Personal 78 führt mit dem Patienten das Rekrutierungsgespräch. Die dafür notwendigen Angaben entnimmt das klinische Personal 78 der Rekrutierungsbeschreibungsinformation 84. Ferner führt das klinische Personal 78 bei einem Einverständnis des Patienten ggf. entsprechend den Angaben der Rekrutierungsbeschreibungsinformation 84 weitere Untersuchungen an dem Patienten durch. Das klinische Personal 78 ermittelt, ob der Patient der Rekrutierung zustimmt, für die Rekrutierung geeignet ist oder für die Rekrutierung ungeeignet ist.

Die Nutzerinterfaceroutine 86 des Patientendatenaufbereitungsservers 28 nimmt von dem klinischen Personal 78 eine Patientenstatusinformation 90 auf, die zumindest beschreibt, ob der Patient der Rekrutierung zustimmt, für die Rekrutierung geeignet ist oder für die Rekrutierung ungeeignet ist. Des Weiteren weist die Patientenstatusinformation 90 Informationen über die weiteren Untersuchungen an dem Patienten auf. Die Patientendatenaufbereitungseinheiten 14 senden die Patientenstatusinformation 90 an den Patientenrekrutierungsserver 16. Die Nutzerinterfaceroutine 60 des Patientenrekrutierungsservers 16 gibt die Patientenstatusinformation 90 an die Rekrutierungsstelle 64 aus.

Der Patientenrekrutierungsserver 16 weist eine Administrationsschnittstelle 92 auf. Über die Administrationsschnittstelle 92 kann ein Administrator 94 die Zugriffmöglichkeiten der mehreren Rekrutierungsstellen 64 und des klinischen Personals 78 konfigurieren. Ferner kann der Administrator 94 andere, dem Fachmann als sinnvoll erscheinende Konfigurationen des Patientenrekrutierungssystems 10 verändern. Die Administrationsschnittstelle 92 kann zumindest teilweise gemeinsam mit der Nutzerinterfaceroutine 60 ausgebildet sein.

## Patentansprüche

1. Patientenrekrutierungssystem (10)
mit zumindest einem Patientenrekrutierungsserver (16),
der zumindest eine Suchroutine (70) aufweist, die dazu vorgesehen ist, Patientendatensätze (32) anhand von Rekrutierungsmerkmalen (66) zu suchen, wobei der Patientenrekrutierungsserver (16) eine Benachrichtigungsroutine (82) aufweist, die dazu vorgesehen ist, wenigstens zu einem mittels der Rekrutierungsmerkmale (66) gefundenen Patientendatensatz (32) eine Rekrutierungsnachricht (76) zu versenden,
wobei die Benachrichtigungsroutine (82) dazu vorgesehen ist, zu anhand der Rekrutierungsmerkmale (66) neu gefundenen Patientendatensätzen (32) automatisch die Rekrutierungsnachricht (76) zu versenden,
wobei der Patientenrekrutierungsserver (16) außerhalb der Intranets (24) jeweiliger Gesundheitsinformationssysteme (20) einer Gesundheitsinstitution angeordnet ist,
wobei das Patientenrekrutierungssystem (10) mehrere verteilt angeordnete Patientendatenaufbereitungseinheiten (14) aufweist, die in zumindest einem Betriebszustand jeweils Patientendatensätze (22) von wenigstens einem Gesundheitsinformationssystem (20) empfangen,
wobei die Patientendatenaufbereitungseinheiten (14) jeweils zumindest eine Pseudonymisierungsroutine (34) aufweisen, die dazu vorgesehen ist, von Gesundheitsinformationssystemen (20) empfangene Patientendatensätze (22) zu pseudonymisieren,
wobei die durchsuchten Patientendatensätze (32) als pseudonymisierte Patientendatensätze (32) ausgebildet sind,
wobei die Patientendatenaufbereitungseinheiten (14) jeweils zumindest eine Depseudonymisierungsroutine (88) aufweisen, die dazu vorgesehen sind, zumindest eine Patientenidentifikationsinformation (38) zu einer Pseudonyminformation (36) der pseudonymisierten Patientendatensätze (32) zu bestimmen, wobei der Patientenrekrutierungsserver (16) an einem anderen Ort angeordnet ist als die Patientendatenaufbereitungseinheiten (14),
wobei jede der Patientendatenaufbereitungseinheiten (14) in einem lokalen Netz und/oder einem Intranet (24), der Gesundheitsinstitution angeordnet ist, wobei die Patientendatenaufbereitungseinheiten (14) und der Patientenrekrutierungsserver (16) über eine verschlüsselte Verbindung über das Internet (58) miteinander verbunden sind,
wobei technische Vorkehrungen vorgesehen sind, die sicherstellen, dass Patientendaten und Patientendatensätze (22), welche Informationen über einen in der Gesundheitsinstitution zumindest untersuchten Patienten aufweisen oder welche Teile von elektronischen Patientenakten eines der Gesundheitsinformationssysteme (20) umfassen, sowie auch die pseudonymisierten Patientendatensätze (32) nicht das lokale Netz der Gesundheitsinstitution und nicht das Intranet (24) der Gesundheitsinstitution verlassen können,
wobei die Patientendatenaufbereitungseinheiten (14) jeweils zumindest einen Pseudonymverwaltungsserver (26) aufweisen, der in zumindest einem Betriebszustand die Pseudonyminformationen (36) und die dazugehörigen Patientenidentifikationsinformationen (38) speichert,
wobei die Pseudonymverwaltungsserver (26) in zumindest einem Betriebszustand die pseudonymisierten Patientendatensätze (32) versenden,
wobei Patientendatenaufbereitungsserver (28) der Patientendatenaufbereitungseinheiten (14) in zumindest einem Betriebszustand die pseudonymisierten Patientendatensätze (32) von den Pseudonymverwaltungsservern (26) empfangen,
wobei die pseudonymisierten Patientendatensätze (32) in einer Datenbank (56) des Patientendatenaufbereitungsservers (28) gespeichert werden,
und wobei die Pseudonymverwaltungsserver (26) und die Patientendatenaufbereitungseinheiten (14) nur über das Intranet (24) des jeweiligen Gesundheitsinformationssystems (20) kommunizieren.

2. Patientenrekrutierungssystem (10) nach Anspruch 1, **dadurch gekennzeichnet, dass** die Suchroutine (70) dazu vorgesehen ist, die Patientendatensätze (32) wiederholt anhand der Rekrutierungsmerkmale (66) zu durchsuchen.

3. Patientenrekrutierungssystem (10) nach Anspruch 1 oder 2, **dadurch gekennzeichnet, dass** die Rekrutierungsnachricht (76) zumindest die Pseudonyminformationen (36) und/oder die Patientenidentifikationsinformationen (38) aufweist.

4. Patientenrekrutierungssystem (10) nach Anspruch 1 oder 2, **dadurch gekennzeichnet, dass** die Patientendatenaufbereitungseinheiten (14) dazu vorgesehen sind, zumindest die Patientenidentifikationsinformation (38) an klinisches Personal (78) auszugeben.

5. Patientenrekrutierungssystem (10) nach Anspruch 1, **dadurch gekennzeichnet, dass** die Patientendatenaufbereitungseinheiten (14) jeweils zumindest eine Nutzerinterfaceroutine (86) aufweisen, die dazu vorgesehen sind, zumindest einen der Patientendatensätze (22, 32) zumindest teilweise an das klinische Personal (62) auszugeben.

6. Patientenrekrutierungssystem (10) nach einem der vorherigen Ansprüche, **dadurch gekennzeichnet, dass** die Patientendatenaufbereitungseinheiten (14) dazu vorgesehen sind, zumindest eine Patientenstatusinformation (90) von dem klinischen Personal (78) aufzunehmen.

7. Patientenrekrutierungssystem (10) nach Anspruch 1, **dadurch gekennzeichnet, dass** die Patientendatenaufbereitungseinheiten (14) jeweils zumindest eine Ontologieroutine (42) aufweisen, die dazu vorgesehen sind, zumindest eine Ontologie der Patientendatensätze (22) zu vereinheitlichen.

8. Patientenrekrutierungssystem (10) nach Anspruch 1, **dadurch gekennzeichnet, dass** die Patientendatenaufbereitungseinheiten (14) jeweils zumindest eine Datenbank (56) aufweisen, die dazu vorgesehen ist, die pseudonymisierten Patientendatensätze (32) zu speichern.

9. Patientenrekrutierungssystem (10) nach einem der vorhergehenden Ansprüche, **dadurch gekennzeichnet, dass** der Patientenrekrutierungsserver (16) zumindest eine Nutzerinterfaceroutine (60) aufweist, die dazu vorgesehen ist, zumindest ein Suchergebnis (74) der Suchroutine (70) an eine Rekrutierungsstelle (64) auszugeben.

10. Patientenrekrutierungssystem (10) nach Anspruch 9, **dadurch gekennzeichnet, dass** die Nutzerinterfaceroutine (60) des Patientenrekrutierungsservers (16) dazu vorgesehen ist, eine Rekrutierungsinformation (62) von der Rekrutierungsstelle (64) aufzunehmen.

11. Patientenrekrutierungssystem (10) nach den Ansprüchen 1 und 10, **dadurch gekennzeichnet, dass** die Patientendatenaufbereitungseinheiten (14) dazu vorgesehen sind, die pseudonymisierten Patientendatensätze (32) anhand von durch die Rekrutierungsinformation (62) verknüpften Rekrutierungsmerkmalen (66) zu suchen.

12. Patientenrekrutierungssystem (10) nach den Ansprüchen 9 und 11, **dadurch gekennzeichnet, dass** die Nutzerinterfaceroutine (60) des Patientenrekrutierungsservers (16) dazu vorgesehen ist, eine Anzahl von gefundenen Patientendatensätzen (32) für einzelne der verknüpften Rekrutierungsmerkmale (66) auszugeben.

13. Patientenrekrutierungssystem (10) nach Anspruch 9, **dadurch gekennzeichnet, dass** die Nutzerinterfaceroutine (60) des Patientenrekrutierungsservers (16) dazu vorgesehen ist, eine Anzahl von gefundenen Patientendatensätzen (32), die von einem der Gesundheitsinformationssysteme (20) stammen, auszugeben.

14. Patientenrekrutierungssystem (10) nach einem der vorhergehenden Ansprüche, **dadurch gekennzeichnet, dass** die Patientendatensätze (22, 32) biomedizinische Daten, Medizinproduktdaten, Labordaten, Personendaten und/oder Sensordaten aufweisen.

15. Patientenrekrutierungsserver (16) für ein Patientenrekrutierungssystem (10) nach einem der vorhergehenden Ansprüche.

16. Patientendatenaufbereitungseinheit (14) für ein Patientenrekrutierungssystem (10) nach Anspruch 1.

17. Patientenrekrutierungsverfahren mit einem Patientenrekrutierungssystem (10), nach Anspruch 1, wobei
- Patientendatensätze (32) anhand von Rekrutierungsmerkmalen (66) durchsucht werden und
- eine Rekrutierungsnachricht (76) versandt wird, wenn mittels der Rekrutierungsmerkmale (66) zumindest ein Patientendatensatz (32) gefunden wird - die Benachrichtigungsroutine (82) zu anhand der Rekrutierungsmerkmale (66) neu gefundenen Patientendatensätzen (32) automatisch die Rekrutierungsnachricht (76) versendet,
wobei der Patientenrekrutierungsserver (16) außerhalb der Intranets (24) jeweiliger Gesundheitsinformationssysteme (20) einer Gesundheitsinstitution angeordnet ist,
wobei das Patientenrekrutierungssystem (10) mehrere verteilt angeordnete Patientendatenaufbereitungseinheiten (14) aufweist, die in zumindest einem Betriebszustand jeweils Patientendatensätze (22) von wenigstens einem Gesundheitsinformationssystem (20) empfangen,
wobei die Patientendatenaufbereitungseinheiten (14) jeweils zumindest eine Pseudonymisierungsroutine (34) aufweisen, mittels der von Gesundheitsinformationssystemen (20) empfangene Patientendatensätze (22) pseudonymisiert werden,
wobei die durchsuchten Patientendatensätze (32) als pseudonymisierte Patientendatensätze (32) ausgebildet sind,
wobei die Patientendatenaufbereitungseinheiten (14) jeweils zumindest eine Depseudonymisierungsroutine (88) aufweisen, mittels der zumindest eine Patientenidentifikationsinformation (38) zu einer Pseudonyminformation (36) der pseudonymisierten Patientendatensätze (32) bestimmt wird,
wobei der Patientenrekrutierungsserver (16) an einem anderen Ort angeordnet ist als die Patientendatenaufbereitungseinheiten (14),
wobei jede der Patientendatenaufbereitungseinheiten (14) in einem lokalen Netz und/oder einem Intranet (24) der Gesundheitsinstitution angeordnet ist, wobei die Patientendatenaufbereitungseinheiten (14) und der Patientenrekrutierungsserver (16) über eine verschlüsselte Verbindung über das Internet (58) miteinander verbunden sind,
wobei der Patientenrekrutierungsserver (16) zumindest eine Nutzerinterfaceroutine (60) aufweist, von der Rekrutierungsinformationen (62) von einer Rekrutierungsstelle (64) aufgenommen werden und von der zumindest ein Suchergebnis (74) der Suchroutine (70) an eine Rekrutierungsstelle (64) ausgegeben wird, wobei durch technische Vorkehrungen sichergestellt wird, dass Patientendaten und Patientendatensätze (22), welche Informationen über einen in der Gesundheitsinstitution zumindest untersuchten Patienten aufweisen oder welche Teile von elektronischen Patientenakten eines der Gesundheitsinformationssysteme (20) umfassen, sowie auch die pseudonymisierten Patientendatensätze (32) nicht das lokale Netz der Gesundheitsinstitution und nicht das Intranet (24) der Gesundheitsinstitution verlassen können,
wobei die Patientendatenaufbereitungseinheiten (14) jeweils zumindest einen Pseudonymverwaltungsserver (26) aufweisen, von dem in zumindest einem Betriebszustand die Pseudonyminformationen (36) und die dazugehörigen Patientenidentifikationsinformationen (38) gespeichert werden,
wobei von den Pseudonymverwaltungsserver (26) in zumindest einem Betriebszustand die pseudonymisierten Patientendatensätze (32) versendet werden,
wobei von Patientendatenaufbereitungsservern (28) der Patientendatenaufbereitungseinheiten (14) in zumindest einem Betriebszustand die pseudonymisierten Patientendatensätze (32) von den Pseudonymverwaltungsservern (26) empfangen werden,
und wobei die pseudonymisierten Patientendatensätze (32) in einer Datenbank (56) des Patientendatenaufbereitungsservers (28) gespeichert werden,
und wobei die Pseudonymverwaltungsserver (26) und die Patientendatenaufbereitungseinheiten (14) nur über das Intranet (24) des jeweiligen Gesundheitsinformationssystems (20) kommunizieren.

## Claims

1. Patient recruitment system (10),
with at least one patient recruitment server (16),
comprising at least one search routine (70) that is configured to search for patient data records (32) on the basis of recruitment characteristics (66),
wherein the patient recruitment server (16) comprises a notification routine (82) that is configured to dispatch a recruitment notification (76) concerning at least one patient data record (32) found on the basis of the recruitment characteristics (66), wherein the notification routine (82) is configured to automatically dispatch the recruitment notification (76) concerning patient data records (32) newly found on the basis of the recruitment characteristics (66),
wherein the patient recruitment server (16) is arranged outside of intranets (24) of respective healthcare information systems (20) of a healthcare institution, wherein the patient recruitment system (10) comprises a plurality of distributedly located patient data processing units (14) which, in at least one operation state, respectively receive patient data records (22) from at least one healthcare information system (20),
wherein the patient data processing units (14) respectively comprise at least one pseudonymization routine (34), which is configured to pseudonymize patient data records (22) received from healthcare information systems (20),
wherein the searched patient data records (32) are realized as pseudonymized patient data records (32),
wherein the patient data processing units (14) respectively comprise at least one depseudonymization routine (88), said depseudonymization routines (88) being configured to determine at least one patient identification information (38) that corresponds to a pseudonym information (36) of the pseudonymized patient data records (32),
wherein the patient recruitment server (16) is located in a place different than the patient data processing units (14),
wherein each of the patient data processing units (14) is arranged in a local network and/or an intranet (24) of the healthcare institution,
wherein the patient data processing units (14) and the patient recruitment server (16) are connected by an encoded connection via the Internet (58), wherein technical precautions are provided, ensuring that neither patient data and patient data records (22) which contain information regarding a patient who was at least examined in the healthcare institution or which comprise portions of electronic health records of one of the healthcare information systems (20) nor the pseudonymized patient data records (32) can leave the local network of the healthcare institution or the intranet (24) of the healthcare institution,
wherein the patient data processing units (14) respectively comprise at least one pseudonym administration server (26) which, in at least one operation state, stores the pseudonym information (36) and the corresponding patient identification information (38),
wherein, in at least one operation state, the pseudonym administration servers (26) dispatch the pseudonymized patient data records (32), wherein, in at least one operation state, patient data processing servers (28) of the patient data processing units (14) receive the pseudonymized patient data records (32) from the pseudonym administration servers (26),
wherein the pseudonymized patient data records (32) are stored in a database (56) of the patient data processing server (28), and
wherein the pseudonym administration server (26) and the patient data processing units (14) communicate only via the intranet (24) of the respective healthcare information system (20).

2. Patient recruitment system (10) according to claim 1,
**characterised in that** the search routine (70) is configured to repeatedly search the patient data records (32) on the basis of the recruitment characteristics (66).

3. Patient recruitment system (10) according to claim 1 or 2,
**characterised in that** the recruitment notification (76) comprises at least the pseudonym information (36) and/or the patient identification information (38).

4. Patient recruitment system (10) according to claim 1 or 2,
**characterised in that** the patient data processing units (14) are configured to output at least the patient identification information (38) to healthcare staff (78).

5. Patient recruitment system (10) according to claim 1,
**characterised in that** the patient data processing units (14) each have at least one user interface routine (86), the user interface routines (86) being configured to output at least one of the patient data records (22, 32) at least partially to the healthcare staff (62).

6. Patient recruitment system (10) according to one of the preceding claims,
**characterised in that** the patient data processing units (14) are configured to collect at least one patient status information (90) from the healthcare staff (78).

7. Patient recruitment system (10) according to claim 1,
**characterised in that** the patient data processing units (14) each comprise at least one ontology routine (42), the ontology routines (42) being configured to standardize at least an ontology of the patient data records (22).

8. Patient recruitment system (10) according to claim 1,
**characterised in that** the patient data processing units (14) each comprise at least one database (56) configured to store the pseudonymized patient data records (32).

9. Patient recruitment system (10) according to one of the preceding claims,
**characterised in that** the patient recruitment server (16) comprises at least one user interface routine (60), which is configured to output at least one search result (74) of the search routine (70) to a recruitment point (64).

10. Patient recruitment system (10) according to claim 9,
**characterised in that** the user interface routine (60) of the patient recruitment server (16) is configured to collect a recruitment information (62) from the recruitment point (64).

11. Patient recruitment system (10) according to claims 1 and 10,
**characterised in that** the patient data processing units (14) are configured to search for the pseudonymized patient data records (32) on the basis of recruitment characteristics (66) which are linked by the recruitment information (62).

12. Patient recruitment system (10) according to claims 9 and 11,
**characterised in that** the user interface routine (60) of the patient recruitment server (16) is configured to output a number of found patient data records (32) for respective ones of the linked recruitment characteristics (66).

13. Patient recruitment system (10) according to claim 9,
**characterised in that** the user interface routine (60) of the patient recruitment server (16) is configured to output a number of found patient data records (32) coming from one of the healthcare information systems (20).

14. Patient recruitment system (10) according to one of the preceding claims,
**characterised in that** the patient data records (22, 32) comprise biomedical data, medical product data, laboratory data, personal data and/or sensor data.

15. Patient recruitment server (16) for a patient recruitment system (10) according to one of the preceding claims.

16. Patient data processing unit (14) for a patient recruitment system (10) according to claim 1.

17. Patient recruitment method with a patient recruitment system (10) according to claim 1, wherein
- patient data records (32) are searched on the basis of recruitment characteristics (66) and
- a recruitment notification (76) is dispatched if, on the basis of the recruitment characteristics (66), at least one patient data record (32) is found, wherein the notification routine (82) automatically dispatches the recruitment notification (76) concerning patient data records (32) newly found on the basis of the recruitment characteristics (66),
wherein the patient recruitment server (16) is located outside the intranets (24) of respective healthcare information systems (20) of a healthcare institution, wherein the patient recruitment system (10) comprises a plurality of distributedly located patient data processing units (14) which, in at least one operation state, respectively receive patient data records (22) from at least one healthcare information system (20),
wherein the patient data processing units (14) respectively comprise at least one pseudonymization routine (34), by means of which patient data records (22) received from healthcare information systems (20) are pseudonymized,
wherein the searched patient data records (32) are realized as pseudonymized patient data records (32),
wherein the patient data processing units (14) respectively comprise at least one depseudonymization routine (88), by which at least one patient identification information (38) is determined that corresponds to a pseudonym information (36) of the pseudonymized patient data records (32),
wherein the patient recruitment server (16) is located in a place different than the patient data processing units (14),
wherein each of the patient data processing units (14) is arranged in a local network and/or an intranet (24) of the healthcare institution,
wherein the patient data processing units (14) and the patient recruitment server (16) are connected by an encoded connection via the Internet (58),
wherein the patient recruitment server (16) comprises at least one user interface routine (60), by which recruitment information (62) is collected from a recruitment point (64) and by which at least one search result (74) of the search routine (70) is output to a recruitment point (64),
wherein it is ensured by technical precautions that neither patient data and patient data records (22), which contain information regarding a patient who was at least examined in the healthcare institution or which comprise portions of electronic health records of one of the healthcare information systems (20), nor the pseudonymized patient data records (32) can leave the local network of the healthcare institution or the intranet (24) of the healthcare institution,
wherein the patient data processing units (14) respectively comprise at least one pseudonym administration server (26) by which, in at least one operation state, the pseudonym information (36) and the corresponding patient identification information (38) are stored,
wherein, in at least one operation state, the pseudonymized patient data records (32) are dispatched by the pseudonym administration servers (26),
wherein, in at least one operation state, the pseudonymized patient data records (32) are received from the pseudonym administration servers (26) by patient data processing servers (28) of the patient data processing units (14), and
wherein the pseudonymized patient data records (32) are stored in a database (56) of the patient data processing server (28), and
wherein the pseudonym administration servers (26) and the patient data processing units (14) communicate only via the intranet (24) of the respective healthcare information system (20).

## Revendications

1. Système de recrutement de patients (10),
avec au moins un serveur informatique de recrutement de patients (16), comprenant au moins une routine de recherche (70) qui est prévue à chercher des jeux de données de patients (32) sur la base de caractéristiques de recrutement (66),
le serveur informatique de recrutement de patients (16) comprenant une routine de notification (82) qui est prévue à envoyer une notification de recrutement (76) concernant au moins un jeu de données de patient (32) trouvé en utilisant les caractéristiques de recrutement (66),
la routine de notification (82) étant prévue à envoyer automatiquement la notification de recrutement (76) concernant des jeux de données de patients (32) nouvellement trouvés en utilisant les caractéristiques de recrutement (66),
le serveur informatique de recrutement de patients (16) étant localisé en dehors des intranets (24) de systèmes d'information-santé (20) respectifs d'une institution de santé,
le système de recrutement de patients (10) comprenant plusieurs unités de traitement de données de patients (14) distribuées qui, dans au moins un état d'opération, respectivement reçoivent des jeux de données de patients (22) d'au moins un système d'information-santé (20),
les unités de traitement de données de patients (14) respectivement comprenant au moins une routine de pseudonymisation (34) qui est prévue à pseudonymiser des jeux de données de patients (22) reçus de systèmes d'information-santé (20), où les jeux de données de patients fouillés (32) sont réalisés comme des jeux de données de patients pseudonymisés (32),
où les unités de traitement de données de patients (14) comprennent respectivement au moins une routine de dépseudonymisation (88), lesdites routines de dépseudonymisation (88) étant prévues à déterminer au moins une information d'identification-patient (38) qui corresponde à une information de pseudonyme (36) des jeux de données de patients pseudonymisés (32),
où le serveur informatique de recrutement de patients (16) est localisé à un autre endroit que les unités de traitement de données de patients (14),
où chacune des unités de traitement de données de patients (14) est localisée dans un réseau local et/ou dans un intranet (24) de l'institution de santé,
où les unités de traitement de données de patients (14) et le serveur informatique de recrutement de patients (16) sont raccordés par une liaison encodée via l'internet (58),
où des préventions techniques sont implémentées, qui assurent que des données de patients et des jeux de données de patients (22) comprenant des informations d'un patient au moins examiné dans l'institution de santé ou qui comprennent des parties de dossiers médicaux électroniques d'un des systèmes d'information-santé (20), aussi que les jeux de données de patients pseudonymisés (32), ne puissent pas sortir ni du réseau local de l'institution de santé ni de l'intranet (24) de l'institution de santé,
où chacune des unités de traitement de données de patients (14) comporte au moins un serveur informatique d'administration de pseudonymes (26) qui, dans au moins un état d'opération, stocke les informations de pseudonyme (36) et les informations d'identification-patients (38) allouées,
où dans au moins un état d'opération les serveurs informatiques d'administration de pseudonymes (26) envoient les jeux de données de patients pseudonymisés (32),
où dans au moins un état d'opération des serveurs informatiques de traitement de données de patients (28) des unités de traitement de données de patients (14) reçoivent les jeux de données de patients pseudonymisés (32) des serveurs informatiques d'administration de pseudonymes (26),
où les jeux de données de patients pseudonymisés (32) sont stockés dans une base de données (56) du serveur informatique de traitement de données de patients (28), et
où les serveurs informatiques d'administration de pseudonymes (26) et les unités de traitement de données de patients (14) communiquent seulement via l'intranet (24) du système d'information-santé (20) respectif.

2. Système de recrutement de patients (10) selon la revendication 1,
**caractérisé en ce que** la routine de recherche (70) est prévue à fouiller les jeux de données de patients (32) itérativement sur la base des caractéristiques de recrutement (66).

3. Système de recrutement de patients (10) selon la revendication 1 ou 2,
**caractérisé en ce que** la notification de recrutement (76) comporte au moins les informations de pseudonyme (36) et/ou les informations d'identification-patient (38).

4. Système de recrutement de patients (10) selon la revendication 1 ou 2,
**caractérisé en ce que** les unités de traitement de données de patients (14) sont prévues à afficher au moins l'information d'identification-patient (38) à un personnel clinique (78).

5. Système de recrutement de patients (10) selon la revendication 1,
**caractérisé en ce que** chacune des unités de traitement de données de patients (14) comprend au moins une routine d'interface utilisateur (86), lesdites routines d'interface utilisateur (86) étant prévues à afficher au moins un des jeux de données de patients (22, 32) au moins partiellement au personnel clinique (62).

6. Système de recrutement de patients (10) selon l'une des revendications précédentes,
**caractérisé en ce que** les unités de traitement de données de patients (14) sont prévues à recorder au moins une information d'état de patient (90) du personnel clinique (78).

7. Système de recrutement de patients (10) selon la revendication 1,
**caractérisé en ce que** chacune des unités de traitement de données de patients (14) comprend au moins une routine d'ontologie (42) qui est prévue à uniformiser au moins une ontologie des jeux de données de patients (22).

8. Système de recrutement de patients (10) selon la revendication 1,
**caractérisé en ce que** chacune des unités de traitement de données de patients (14) comprend au moins une base de données (56), qui est prévue à stocker les jeux de données de patients pseudonymisés (32).

9. Système de recrutement de patients (10) selon l'une des revendications précédentes,
**caractérisé en ce que** le serveur informatique de recrutement de patients (16) comprend au moins une routine d'interface utilisateur (60) qui est prévue à afficher au moins un résultat de recherche (74) de la routine de recherche (70) à un point de recrutement (64).

10. Système de recrutement de patients (10) selon la revendication 9,
**caractérisé en ce que** la routine d'interface utilisateur (60) du serveur informatique de recrutement de patients (16) est prévue à recorder une information de recrutement (62) du point de recrutement (64).

11. Système de recrutement de patients (10) selon les revendications 1 et 10,
**caractérisé en ce que** les unités de traitement de données de patients (14) sont prévues à chercher les jeux de données de patients pseudonymisés (32) moyennant des caractéristiques de recrutement (66) reliées par l'information de recrutement (62).

12. Système de recrutement de patients (10) selon les revendications 9 et 11,
**caractérisé en ce que** la routine d'interface utilisateur (60) du serveur informatique de recrutement de patients (16) est prévue à afficher un nombre de jeux de données de patients trouvés (32) pour quelques caractéristiques de recrutement particulières (66).

13. Système de recrutement de patients (10) selon la revendication 9,
**caractérisé en ce que** la routine d'interface utilisateur (60) du serveur informatique de recrutement de patients (16) est prévue à afficher un nombre de jeux de données de patients trouvés (32) provenant de l'un des systèmes d'information-santé (20).

14. Système de recrutement de patients (10) selon l'une des revendications précédentes,
**caractérisé en ce que** les jeux de données de patients (22, 32) comportent de données biomédicales, de données de produits médicaux, de données de laboratoire, de données personnelles et/ou de données de capteur.

15. Serveur informatique de recrutement de patients (16) pour un système de recrutement de patients (10) selon l'une des revendications précédentes.

16. Unité de traitement de données de patients (14) pour un système de recrutement de patients (10) selon la revendication 1.

17. Procédé de recrutement de patients avec un système de recrutement de patients selon la revendication 1, où
- des jeux de données de patients (32) sont fouillés en utilisant des caractéristiques de recrutement (66) et
- une notification de recrutement (76) est envoyé si au moins un jeu de données de patient (32) est trouvé moyennant les caractéristiques de recrutement (66) et la routine de notification (82) automatiquement envoie la notification de recrutement (76) concernant des jeux de données de patients (32) nouvellement trouvés moyennant les caractéristiques de recrutement (66),
où le serveur informatique de recrutement de patients (16) est localisé en dehors de l'intranet (24) de systèmes d'information-santé (20) respectifs d'une institution de santé,
où le système de recrutement de patients (10) comprend plusieurs unités de traitement de données de patients (14) distribuées qui, dans au moins un état d'opération, respectivement reçoivent des jeux de données de patients (22) d'au moins un système d'information-santé (20),
où les unités de traitement de données de patients (14) respectivement comprennent au moins une routine de pseudonymisation (34), moyennant laquelle des jeux de données de patients (22) reçus de systèmes d'information-santé (20) sont pseudonymisés,
où les jeux de données de patients fouillés (32) sont réalisés comme des jeux de données de patients pseudonymisés (32),
où les unités de traitement de données de patients (14) comprennent respectivement au moins une routine de dépseudonymisation (88), moyennant laquelle au moins une information d'identification-patient (38) est déterminée qui corresponde à une information de pseudonyme (36) des jeux de données de patients pseudonymisés (32),
où le serveur informatique de recrutement de patients (16) est localisé dans un autre endroit que les unités de traitement de données de patients (14),
où chacune des unités de traitement de données de patients (14) est localisée dans un réseau local et/ou un intranet (24) de l'institution de santé,
où les unités de traitement de données de patients (14) et le serveur de recrutement de patients (16) sont raccordés par une liaison encodée via l'internet (58), où le serveur informatique de recrutement de patients (16) comporte au moins une routine d'interface utilisateur (60) par laquelle des informations de recrutement (62) sont obtenues d'un point de recrutement (64) et par laquelle au moins un résultat de recherche (74) de la routine de recherche (70) est affiché à un point de recrutement (64),
où il est assuré par des préventions techniques que des données de patients et des jeux de données de patients (22), qui contiennent des informations d'un patient au moins examiné dans l'institution de santé ou qui comprennent des parties de dossiers médicaux électroniques d'un des systèmes d'information-santé (20), aussi que des jeux de données de patients pseudonymisés (32), ne puissent pas sortir ni du réseau local de l'institution de santé ni de l'intranet (24) de l'institution de santé,
où chacune des unités de traitement de données de patients (14) comprend au moins un serveur informatique d'administration de pseudonymes (26), par le biais duquel, dans au moins un état d'opération, les informations de pseudonyme (36) et les informations d'identification-patient correspondantes (38) sont stockées,
où dans au moins un état d'opération, par le biais de serveurs informatiques d'administration de pseudonymes (26), les jeux de données de patients pseudonymisés (32) sont envoyés,
où dans au moins un état d'opération, par le biais de serveurs informatiques de traitement de données de patients (28) des unités de traitement de données de patients (14), les jeux de données de patients pseudonymisés (32) sont reçus des serveurs informatiques d'administration de pseudonymes (26), et
où les jeux de données de patients pseudonymisés (32) sont stockés dans une base de données (56) du serveur informatique de traitement de données de patients (28), et
où les serveurs informatiques d'administration de pseudonymes (26) et les unités de traitement de données de patients (14) communiquent seulement via l'intranet (24) du système d'information-santé (20) respectif.
